# EUROPEAN PATENT APPLICATION

(11) **EP 3 184 088 A1**
(43) Date of publication of application: **28.06.2017**
(21) Application number: 16205719.4
(22) Date of filing: 21.12.2016
(51) Int. Cl.: A61F 5/00, A61B 17/122

(54) **SURGICAL DEVICES FOR PERFORMING A BARIATRIC PROCEDURE**

(30) Priority: 22.12.2015 IN 6824CH2015
(71) Applicant: Covidien LP, Mansfield, MA 02048 (US)
(72) Inventor: MURTHY ARAVALLI, Anantha Venkata Varaha Lakshmi Narasimha Srinivasa, 500019 Andhra Pradesh (IN)
(74) Representative: Maschio, Antonio

(57) **Abstract**

A method of performing a vertical sleeve gastrostomy. The method includes positioning a surgical device adjacent a stomach, positioning a first leg of the surgical device on a dorsal side of the stomach, positioning a second leg of the surgical device on a ventral side of the stomach, approximating the first leg with respect to the second leg, and limiting movement between the first leg and the second leg with a closure mechanism.

## Description

### BACKGROUND

### Technical Field

The present disclosure relates to performing a bariatric procedure and surgical devices for use therewith. More particularly, the present disclosure relates to the use of a surgical clip for performing a bariatric surgery (e.g., a vertical sleeve gastrectomy).

### Background of Related Art

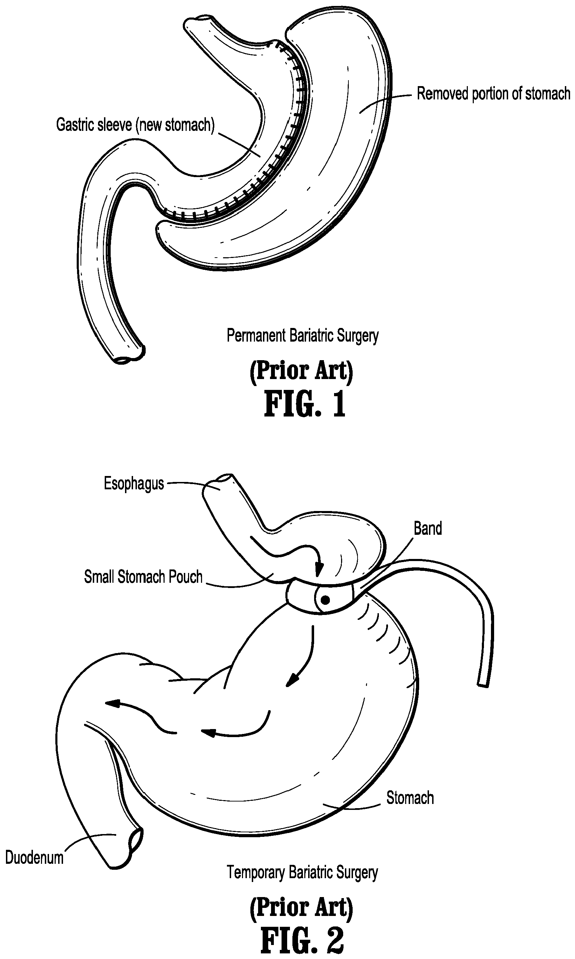

A vertical sleeve gastrectomy is a permanent procedure and is a restrictive form of weight loss surgery in which approximately 85% of the left side of the stomach is removed leaving a cylindrical- or sleeve-shaped stomach with a capacity ranging from about 60 cubic centimeters to about 150 cubic centimeters. A vertical sleeve gastrectomy results in a remodeled stomach that resembles the size and shape of a banana (FIG. 1 schematically illustrates the result of a vertical sleeve gastrectomy). Unlike many other forms of bariatric surgery, the outlet valve and the nerves to the stomach remain intact and, while the stomach is drastically reduced in size, its function is preserved.

A temporary or reversible bariatric surgery may employ a device to block or reduce an amount of stomach volume (as opposed to removal of a portion of the stomach) to limit the flow of food intake from the esophagus. Such devices are typically placed laterally or horizontally on a portion of the stomach (see FIG. 2).

Devices that are typically used for a temporary or reversible bariatric surgery cannot be used for a vertical sleeve gastrostomy due at least in part to its size and/or strength limitations.

In view of the foregoing, a need exists for methods of using a surgical device to perform a temporary or reversible vertical sleeve gastrostomy.

### SUMMARY

The present disclosure relates to a method of performing a vertical sleeve gastrostomy. The method includes positioning a surgical device adjacent a stomach, positioning a first leg of the surgical device on a dorsal side of the stomach, positioning a second leg of the surgical device on a ventral side of the stomach, approximating the first leg with respect to the second leg, and limiting movement between the first leg and the second leg with a closure mechanism.

In disclosed embodiments, approximating the first leg with respect to the second leg includes pivoting the first leg with respect to the second leg.

It is further disclosed that limiting movement between the first leg and the second leg with a closure mechanism includes securing the first leg to the second leg, such as temporarily securing the first leg to the second leg.

The disclosed method also includes adjusting the closure mechanism to change tension acting on tissue between the first leg and the second leg.

In disclosed embodiments, approximating the first leg with respect to the second is done by contacting the first leg and the second leg with at least one hand of a physician.

It is further disclosed that the method includes removing the surgical device from contact with the stomach.

The present disclosure also relates to a a surgical device for performing a vertical sleeve gastrostomy. The surgical device includes a first leg, a second leg, and a closure mechanism. The second leg is pivotally engaged with the first leg. The closure mechanism is disposed in mechanical cooperation with at least one of the first leg and the second leg and is configured to limit movement between the first leg and the second leg. Each of the first leg and the second leg includes a length of between about 8 inches and about 16 inches.

In disclosed embodiments, each of the first leg and the second leg includes a length of about 12 inches.

It is further disclosed that the closure mechanism is configured to be a temporary closure mechanism.

In additional embodiments, the surgical device includes a second closure mechanism disposed in mechanical cooperation with at least one of the first leg and the second leg and is configured to limit movement between the first leg and the second leg.

In disclosed embodiments, the surgical device includes a living hinge disposed between the first leg and the second leg.

### BRIEF DESCRIPTION OF THE FIGURES

Various aspects of the present disclosure are described hereinbelow with reference to the drawings, which are incorporated and constitute a part of this specification, wherein:
FIG. 1 schematically illustrates a patient's resected stomach as a result of a vertical sleeve gastrectomy in accordance with a method of the prior art;
FIG. 2 schematically illustrates a band placed laterally on a portion of a patient's stomach in accordance with a method of the prior art;
FIG. 3 illustrates a surgical device engaged with a portion of a patient's stomach according to embodiments of the present disclosure;
FIGS. 4 and 5 are perspective views of different embodiments of surgical devices of the present disclose in an open position;
FIGS. 6 and 7 are side views of different embodiments of surgical devices of the present disclosure illustrated in the closed position;
FIG. 8 is a perspective view of another surgical device of the present disclosure illustrated in a closed position;
FIG. 9 is a perspective view of yet another surgical device of the present disclosure in a closed position and clamped on a portion of a patient's stomach;
FIG. 10 is a perspective view of still another surgical device in an open position in accordance with embodiments of the present disclosure;
FIG. 11 is a perspective view of a surgical device clamping tissue in accordance with another embodiment of the present disclosure; and
FIG. 12 is a schematic side view of a surgeon's hand clamping a surgical device onto tissue in accordance with embodiments of the present disclosure.

### DETAILED DESCRIPTION

Embodiments of the presently disclosed methods for performing bariatric procedures and surgical devices for use therewith will now be described in detail with reference to the drawings wherein like reference numerals designate identical or corresponding elements in each of the several views. In the description that follows, the term "proximal," will refer to the portion of the surgical device closest to its hinge, and the term "distal" will refer to the portion of the surgical device farthest from its hinge.

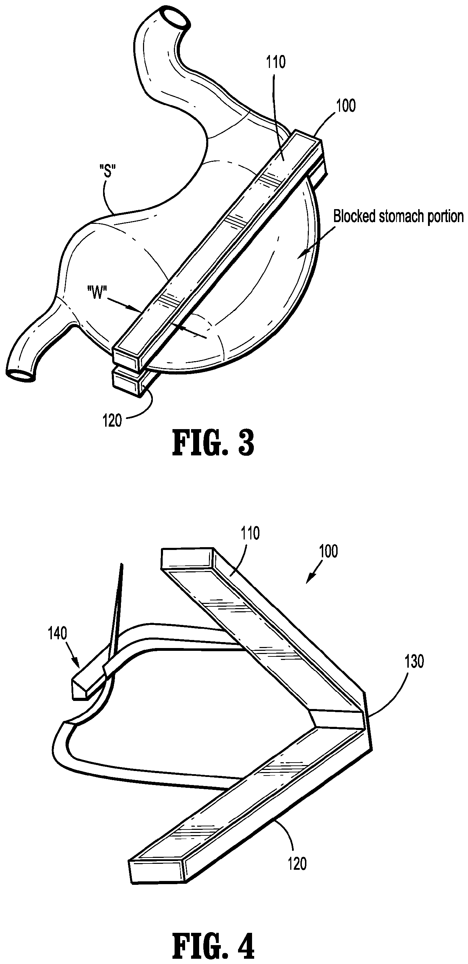

With reference to FIGS. 3-12, various embodiments of surgical devices or surgical clips are illustrated and are generally identified as reference character 100. With particular reference to FIG. 3, surgical device 100 is configured to temporarily clamp onto a portion of a patient's stomach "S" to block off that portion from the flow of food therethrough, for example. In contrast to typical, permanent bariatric procedures (schematically illustrated in FIG. 1), surgical device 100 allows a section of the stomach to be blocked off, without being physically separated from the remainder of the stomach.

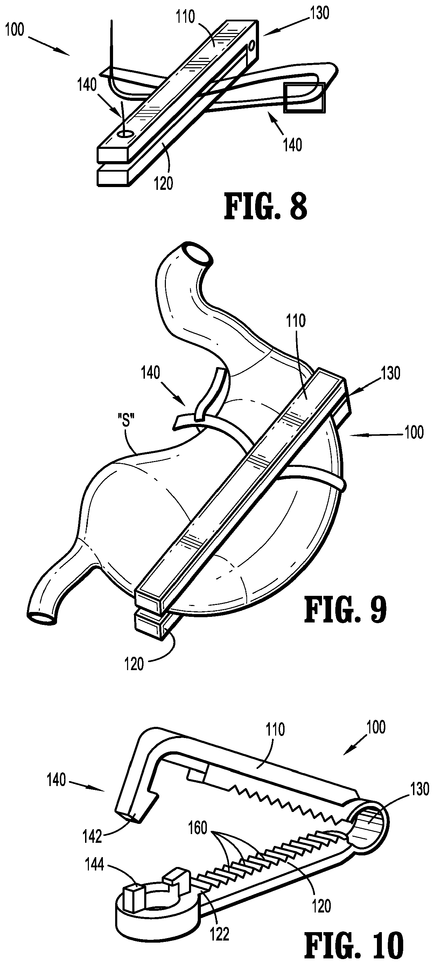

Surgical device 100 generally includes a first leg 110, a second leg 120, a hinge 130, and a closure mechanism 140. First leg 110 engages or is attached to second leg 120 via hinge 130, and closure mechanism 140 helps maintain a desired gap between first leg 110 and second leg 120. As shown in FIGS. 3 and 9, for example, when surgical device 100 is positioned on a portion of the stomach "S," first leg 110 is positioned against a dorsal or upper portion of the stomach "S," and second leg 120 is positioned against a ventral or lower portion of the stomach "S."

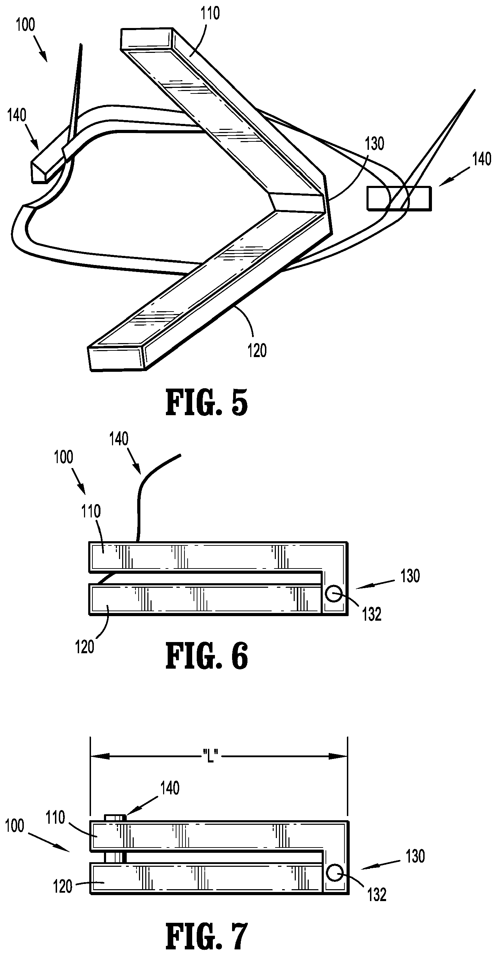

First leg 110 and second leg 120 are movable with respect to each other via hinge 130. As shown in the illustrated embodiments, for example, hinge 130 may be any suitable type of hinge 130 that allows for movement of first leg 110 and/or second leg 120 with respect to the other leg. For instance, in FIGS. 4, 5 and 10, hinge 130 is a living hinge; in FIGS. 6, 7, 11 and 12, hinge 130 includes a pinned hinge including a hinge pin 132 extending through apertures in proximal portions in each of first leg 110 and second leg 120. As can be appreciated, each of the disclosed embodiments of surgical device 100 may include a living hinge, a pinned hinge or another suitable hinge 130. Additionally, other mechanical structures are usable to allow first leg 110 to move (e.g., pivotable movement or non-pivotable movement) with respect to second leg 120.

Closure mechanism 140 of surgical device 100 is configured to help maintain first leg 110 and second leg 120 in an approximated position about tissue (i.e., the stomach "S"). As can be appreciated, compressed tissue that surgical device 100 is clamped upon has the tendency to expand toward its natural position. Closure mechanism 140 helps resists this expansion force of the tissue to help maintain a desired closure gap between first leg 110 and second leg 120.

As shown in the accompanying figures, several types of closure mechanisms 140 are disclosed and usable with surgical device. Further, surgical device 100 may include a first closure mechanism disposed between proximal and distal ends of first leg 110 and second leg 120 (e.g., see FIGS. 4, 5 and 9), and/or may include a second closure mechanism disposed at or near a distal end of first leg 110 and second 120 (e.g., see FIGS. 6, 7 and 10).

First closure mechanism and second closure mechanism, collectively referred to herein as "closure mechanism," may be selected from at least one of several types of devices or mechanisms, such as devices that include adjustment features. For instance, surgical devices 100 of FIGS. 4-6, 8 and 9 include at least one adjustable closure mechanism 140 (e.g., cable, wire, cable tie, ratchet and pawl mechanism, or other adjustable tension mechanism) for helping to maintain the relative positioning of first leg 110 and second leg 120. Surgical device 100 of FIG. 7 includes a pin or screw mechanism to adjust and maintain the distance between first leg 110 and second leg 120. It is further envisioned that closure mechanism 140 can be any other mechanical, pneumatic, hydraulic lock with positive locking, etc.

Surgical device 100 of FIG. 10 includes a two-piece locking mechanism or closure mechanism 140 to maintain the distance between first leg 110 and second leg 120. Here, first leg 110 includes a first portion 142 (e.g., a finger having a ramp) of closure mechanism 140, and second leg 120 includes a second portion 144 (e.g., a slot, cavity, notch, etc.) of closure mechanism 140; first portion 142 of closure mechanism 140 is configured to mechanically engage second portion 144 of closure mechanism 140.

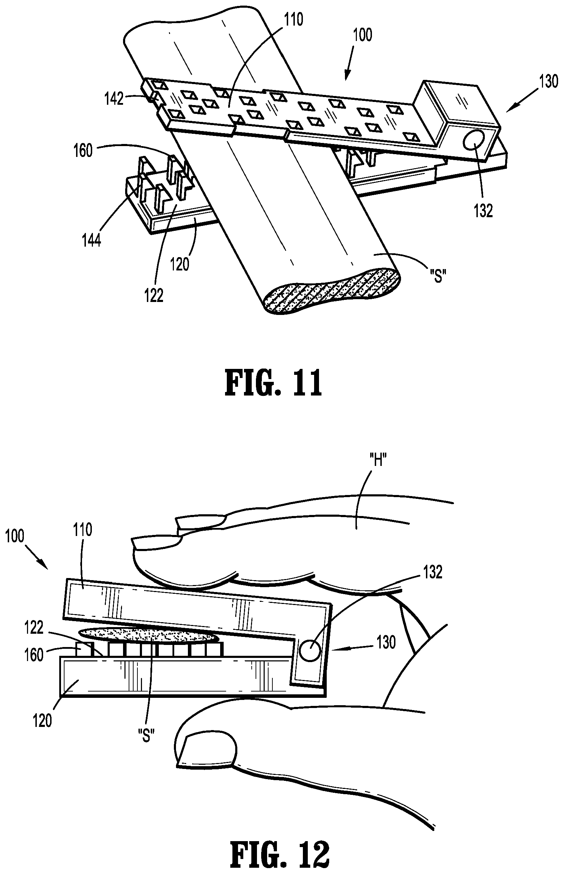

Surgical device 100 of FIG. 11 also includes a two-piece locking mechanism or closure mechanism 140 to maintain the distance between first leg 110 and second leg 120. In this embodiment, first portion 142 of closure mechanism 140 is located on first leg 110 and includes a notch. Second portion 144 of closure mechanism 140 is included on second leg 120 and is a finger configured to engage (e.g., releasably engage) the notch 142 of first leg 110.

As can be appreciated surgical device 100 may include any combination of at least one first closure mechanism and at least one second closure mechanism, and any type of closure mechanism 140 (e.g., adjustable closure mechanism) such as those described herein.

With particular reference to FIGS. 11-13, a tissue-contacting surface 122 of second leg 120 of surgical device 100 of these embodiments, or any of the embodiments disclosed herein, includes a plurality of pins 160 extending therefrom. Pins 160 may be useful to increase the surface area and/or gripping strength of second leg 120 to help ensure a desired grip on tissue. First leg 110 may also include a plurality of pins 160 extending from a tissue-contacting surface 112 thereof. The size, amount and spacing of pins 160 may be different from what is shown in the figures without departing from the scope of the present disclosure.

First leg 110 and second 120 are sufficiently long to enable clamping of an appropriate length of the stomach "S" (e.g., an entire length or essentially an entire length of the stomach "S," as shown in FIGS. 3 and 9, for example). For instance, it is envisioned that each of first leg 110 and second leg 120 includes a length "L" (see FIG. 7) of between about 8 inches and about 16 inches. It is further envisioned that each of first leg 110 and second 1eg 120 is about 12 inches long. Additionally, first leg 110 and second leg 120 may be the same or substantially the same length as each other, or first leg 110 may be longer or shorter than second leg 120.

Additionally, the lateral cross-sectional shape of first leg 110 and/or second 120 may be rectangular, may include at least one rounded edge or corner, etc. Additionally, the lateral cross-sectional shape of first leg 110 and/or second leg 120 may include a taller center portion (i.e., at the middle of the width "W" (see FIG. 3) of the respective leg), and get smaller (either in a stepped, linear or curved configuration) toward lateral edges of the respective leg. Such a configuration may provide increased tissue oxygenation and/or reduced trauma.

Surgical device 100 may be made from an absorbable plastic material, a polymer material (e.g., Radel ® polyphenylsulfone (PPSU)), metal, etc.). Additionally, surgical device 100 may be implantable, absorbable or removable.

Methods of performing a temporary or reversible vertical sleeve gastrostomy using surgical device 100 are also disclosed. Such methods include positioning surgical device 100 adjacent a portion of the stomach "S" (e.g., in a substantially vertical position (as shown in FIGS. 3 and 9) such that the flow of food from the esophagus to the duodenum is not restricted), positioning first leg 110 on an upper or dorsal side of the stomach "S," positioning second leg 120 on a lower or ventral side of the stomach "S," approximating the first leg 110 with respect to the second leg 120 to clamp the tissue of the stomach "S," and securing (e.g., temporarily securing) the first leg 110 and the second leg 120 with closure mechanism 140 to limit movement therebetween. Additionally, a user may adjust the tension of surgical device 100 (e.g., by adjusting closure mechanism 140) to help reduce instances of tissue necrosis, and to improve tissue oxidation level, for instance.

As shown in FIG. 12, it is envisioned that surgical device 100 can be clamped onto tissue with a physician's hand "H" or hands, or a surgeon's hand "H" or hands. Alternatively, a surgical instrument (e.g., a laparoscopic instrument) can be used to clamp surgical device 100 onto tissue.

To remove surgical device 100, a user can remove, loosen or unlock closure mechanism 140, for example, by cutting cord, tie, cable, loosening screw, etc., and then taking surgical device 100 out of contact with the stomach "S," and out of the patient's body.

While the above description contains many specifics, these specifics should not be construed as limitations on the scope of the present disclosure, but merely as illustrations of various embodiments thereof. Therefore, the above description should not be construed as limiting, but merely as exemplifications of various embodiments. Those skilled in the art will envision other modifications within the scope and spirit of the claims appended hereto.

The invention may be described by reference to the following numbered paragraphs:-
1. A method of performing a vertical sleeve gastrostomy, comprising:
   positioning a surgical device adjacent a stomach;
   positioning a first leg of the surgical device on a dorsal side of the stomach;
   positioning a second leg of the surgical device on a ventral side of the stomach;
   approximating the first leg with respect to the second leg; and
   limiting movement between the first leg and the second leg with a closure mechanism.
2. The method according to paragraph 1, wherein approximating the first leg with respect to the second leg includes pivoting the first leg with respect to the second leg.
3. The method according to paragraph 1, wherein limiting movement between the first leg and the second leg with a closure mechanism includes securing the first leg to the second leg.
4. The method according to paragraph 3, wherein securing the first leg to the second leg includes temporarily securing the first leg to the second leg.
5. The method according to paragraph 1, further comprising adjusting the closure mechanism to change tension acting on tissue between the first leg and the second leg.
6. The method according to paragraph 1, wherein approximating the first leg with respect to the second is done by contacting the first leg and the second leg with at least one hand of a physician.
7. The method according to paragraph 1, further comprising removing the surgical device from contact with the stomach.
8. A surgical device for performing a vertical sleeve gastrostomy, comprising:
   a first leg;
   a second leg being pivotally engaged with the first leg; and
   a closure mechanism disposed in mechanical cooperation with at least one of the first leg and the second leg and being configured to limit movement between the first leg and the second leg;
   wherein each of the first leg and the second leg includes a length of between about 8 inches and about 16 inches.
9. The surgical device according to paragraph 8, wherein each of the first leg and the second leg includes a length of about 12 inches.
10. The surgical device according to paragraph 8, wherein the closure mechanism is configured to be a temporary closure mechanism.
11. The surgical device according to paragraph 8, further comprising a second closure mechanism disposed in mechanical cooperation with at least one of the first leg and the second leg and being configured to limit movement between the first leg and the second leg.
12. The surgical device according to paragraph 8, further comprising a living hinge disposed between the first leg and the second leg.

## Claims

1. A surgical device for performing a vertical sleeve gastrostomy, comprising:
a first leg;
a second leg being pivotally engaged with the first leg; and
a closure mechanism disposed in mechanical cooperation with at least one of the first leg and the second leg and being configured to limit movement between the first leg and the second leg;
wherein each of the first leg and the second leg includes a length of between about 8 inches and about 16 inches.

2. The surgical device according to claim 1, wherein each of the first leg and the second leg includes a length of about 12 inches.

3. The surgical device according to claim 1 or claim 2, wherein the closure mechanism is configured to be a temporary closure mechanism.

4. The surgical device according to any preceding claim, further comprising a second closure mechanism disposed in mechanical cooperation with at least one of the first leg and the second leg and being configured to limit movement between the first leg and the second leg.

5. The surgical device according to any preceding claim, further comprising a living hinge disposed between the first leg and the second leg.
